# EUROPEAN PATENT APPLICATION

(11) **EP 4 092 129 A1**
(43) Date of publication of application: **23.11.2022**
(21) Application number: 20913080.6
(22) Date of filing: 17.01.2020
(51) Int. Cl.: C12Q 1/02

(54) **EVALUATION METHOD OF DIFFERENTIATION STATE OF CELLS, AND CELL CULTURE SYSTEM**

(71) Applicant: Hitachi High-Tech Corporation, Minato-ku Tokyo 105-6409 (JP)
(72) Inventor: SHIBUYA, Keisuke, Tokyo 100-8280 (JP); KAWARAI, Masako, Tokyo 105-6409 (JP)
(74) Representative: Strehl Schübel-Hopf & Partner
(86) International application number: PCT/JP2020/001488
(87) International publication number: WO 2021/144956

(57) **Abstract**

A method is provided which noninvasively monitors cells and which can accurately determine the progression of differentiation of the cells. This method, for evaluating the differentiation state of cells during culturing for inducing undifferentiated pluripotent stem cells to differentiate into desired cells, determines the progression of induced differentiation using any of the metabolites of glycolysis or any of the metabolites of the tricarboxylic acid cycle (TCA cycle), the metabolites being selected from among two or more types of amino acids contained in the culture solution or components in the culture solution derived from metabolism of the cells.

## Description

### Technical Field

The present invention relates to, for example, a method for determining the progression of induced differentiation of cells during culturing cells used in regenerative medicine, and a cell culture system.

### Background Art

In regenerative medicine, cells (stem cells) that turn into skin, nerves, bones, blood vessels, and the like are used to restore the functions of tissues and organs that have failed due to illness or injury. Main types of stem cells include somatic stem cells, embryonic stem cells (ES cells), and induced pluripotent stem cells (iPS cells). Somatic stem cells are immature cells contained in trace amounts in bone marrow, fat, blood and the like, and differentiate into certain types of cells. There are hematopoietic stem cells that become blood cells when grown, mesenchymal stem cells that become bone, fat, and the like, neural stem cells that become nerves of the brain, and the like.

The somatic stem cells are characterized in that no rejection occurs because the patient's own cells are collected. The ES cells are established from some of fertilized eggs, and have a function of becoming all the cells of the body. The iPS cells are produced by introducing genes into cells such as skin. Similarly to the ES cells, the iPS cells are considered to become all the cells.

The amount of each of the above-described cells to be collected is small, so undifferentiated stem cells are cultured in vitro, the cells are grown, the cells are induced to differentiate into an appropriate type of cells, and the cells are transplanted into a patient, thereby making it possible to treat a specific disease. The steps of producing cells for preparing cells in vitro are as follows.

Step 1: Separation and purification of collected stem cells
Step 2: Growth and culture of stem cells (increase the number of cells to the required number)
Step 3: Induction of stem cells to differentiate into target cells or tissues
Step 4: Formulation and commercialization of differentiated cells into an appropriate form

The above steps are all performed under aseptic conditions, and are also performed in a cell processing center so as not to be contaminated with other cells. In addition, in each of the above steps, it is necessary to monitor the cell state in order to confirm that the cells are in an appropriate state (the maintenance of the undifferentiation state in step 2, and the progression of differentiation into target cells in step 3). In the determination of the cell state, a staining method with a substance that specifically binds to a specific cell is often used.

For example, since many of the stem cells usually express specific cell surface proteins such as TRA-1-60, TRA-1-81, SSEA3, and SSEA4, the undifferentiation state of the cells can be determined by staining these cells with antibodies against the cell surface proteins. Meanwhile, cell surface markers specifically expressed in adipocytes, osteoblasts, neuronal cells, and the like can be used for cells after differentiation induction.

The state of the cells stained with the cell surface markers is determined by an image of the cells taken by a fluorescence microscope, the number of stained cells obtained by flow cytometry, and the fluorescence intensity of the cells.

In addition, PTL 1 discloses a method for assessing an undifferentiation state of pluripotent stem cells on the basis of changes over time in a variation value of an extracellular metabolite included in a culture medium in which the pluripotent stem cells have been cultured. Furthermore, as extracellular metabolites, the group consisting of L-glutamic acid, L-alanine and ammonia is disclosed.

### Citation List

### Patent Literature

PTL 1: WO 16/052558

### Summary of Invention

### Technical Problem

In applying the method for determining the cell state (mainly the progression of differentiation) by the staining method as described above to the step of preparing cells for treatment purposes, there are the following problems.

Since a substance serving as a marker is attached to the cells for staining, the stained cells cannot be used as a treatment material. In addition, it is desirable that the timing of cell transplantation (the time when the cell state is the best) can be determined after differentiation induction, and for this purpose, it is necessary to monitor the progression of differentiation of the cells over time. In the staining method, since it is necessary to sample the cells multiple times over time, an increase in risk of contamination, labor, time, loss of cells, and the like occur.

Although the method of PTL 1 is a noninvasive method, it is desired to further improve the accuracy of the evaluation index of the differentiation state.

Thus, an object of the present invention is to provide a method which noninvasively monitors cells and which can accurately determine the progression of differentiation of the cells.

### Solution to Problem

In order to solve the above-described problems, the method for evaluating a differentiation state of cells according to the present invention is a method for evaluating the differentiation state of cells during culturing for inducing undifferentiated pluripotent stem cells to differentiate into desired cells, in which the method determines the progression of induced differentiation using any of the metabolites of glycolysis or any of the metabolites of the tricarboxylic acid cycle (TCA cycle), the metabolites being selected from among two or more types of amino acids contained in the culture solution or components in the culture solution derived from metabolism of the cells.

### Advantageous Effects of Invention

According to the present invention, it is possible to provide a method which noninvasively monitors cells and which can accurately determine the progression of differentiation of the cells.

Problems, configurations, and effects other than those described above will be clarified by the description of embodiments below.

### Brief Description of Drawings

[FIG. 1] FIG. 1 is a diagram showing a relationship among culture environment, intracellular metabolism, cell quality, and culture solution components.
[FIG. 2] FIG. 2 is a diagram illustrating a device configuration of a cell culture system.
[FIG. 3] FIG. 3 is a diagram illustrating a device configuration of a cell culture system.
[FIG. 4] FIG. 4 is a diagram showing an example of a culture device.
[FIG. 5] FIG. 5 is a diagram showing an example of an aseptic sampling method.
[FIG. 6] FIG. 6 is a diagram showing a procedure for determining a cell state in offline measurement.
[FIG. 7] FIG. 7 is a diagram showing a procedure for determining a cell state in in-line measurement.
[FIG. 8] FIG. 8 is a diagram illustrating an outline of an intracellular metabolic pathway in animal cells.
[FIG. 9] FIG. 9 is a schematic view showing the principle of intracellular metabolic analysis.
[FIG. 10] FIG. 10 is views showing changes over time in cell morphology during inducing human mesenchymal stem cells to differentiate into adipocytes.
[FIG. 11] FIG. 11 is diagrams showing an increase or decrease of extracellular amino acids in a culture solution during inducing human mesenchymal stem cells to differentiate into adipocytes.
[FIG. 12] FIG. 12 is a diagram showing changes over time in intracellular metabolic reaction rate during inducing human mesenchymal stem cells to differentiate into adipocytes.
[FIG. 13] FIG. 13 is a diagram showing an example of changes over time in index value in a culture solution during inducing human mesenchymal stem cells to differentiate into adipocytes.
[FIG. 14] FIG. 14 is views showing changes over time in cell morphology during inducing human mesenchymal stem cells to differentiate into osteoblasts.
[FIG. 15] FIG. 15 is diagrams showing an increase or decrease of extracellular amino acids in a culture solution during inducing human mesenchymal stem cells to differentiate into osteoblasts.
[FIG. 16] FIG. 16 is a diagram showing changes over time in intracellular metabolic reaction rate during inducing human mesenchymal stem cells to differentiate into osteoblasts.
[FIG. 17] FIG. 17 is a diagram showing an example of changes over time in index value in a culture solution during inducing human mesenchymal stem cells to differentiate into osteoblasts.
[FIG. 18] FIG. 18 is views showing changes over time in cell morphology during inducing human mesenchymal stem cells to differentiate into adipocytes.
[FIG. 19] FIG. 19 is diagrams showing an increase or decrease of extracellular amino acids in a culture solution during inducing human mesenchymal stem cells to differentiate into neuronal cells.
[FIG. 20] FIG. 20 is a diagram showing changes over time in intracellular metabolic reaction rate during inducing human mesenchymal stem cells to differentiate into neuronal cells.

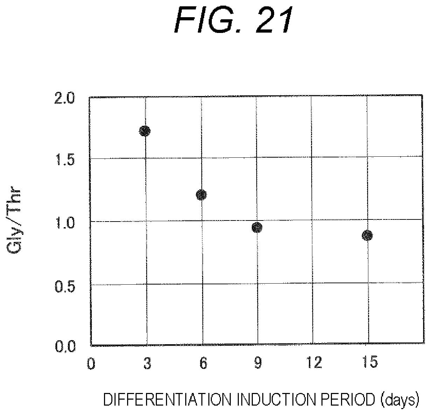

[FIG. 21] FIG. 21 is a diagram showing an example of changes over time in index value in a culture solution during inducing human mesenchymal stem cells to differentiate into neuronal cells.
[FIG. 22] FIG. 22 is views showing changes over time in cell morphology during culturing human mesenchymal stem cells while maintaining an undifferentiation state.
[FIG. 23] FIG. 23 is diagrams showing changes over time in intracellular metabolic reaction rate during culturing human mesenchymal stem cells while maintaining an undifferentiation state.
[FIG. 24] FIG. 24 is a diagram showing changes over time in index value in adipocytes in a case where the undifferentiation state is maintained in human mesenchymal stem cells.
[FIG. 25] FIG. 25 is a diagram showing changes over time in index value in osteoblasts in a case where the undifferentiation state is maintained in human mesenchymal stem cells.
[FIG. 26] FIG. 26 is a diagram showing changes over time in index value in adipocytes in a case where the undifferentiation state is maintained in human mesenchymal stem cells.

### Description of Embodiments

In the invention according to an embodiment of the present invention, a correlation between the cell state and the extracellular culture solution components and a substance as an index for determination is derived from a correlation between the intracellular metabolic reaction rate and the cell state and a correlation between the intracellular metabolic reaction rate and the extracellular culture solution components, the cells are noninvasively monitored by monitoring the derived index component in the extracellular culture solution, and the progression of differentiation of the cells is determined. According to the method for evaluating the progression of induced differentiation of cells, the degree of differentiation of cells can be measured temporally and noninvasively with respect to the cells. Thus, it can be used for quality control in the culturing step, or used for determination of appropriate time for cell transplantation in cell therapy.

Hereinafter, the present invention will be described in detail.

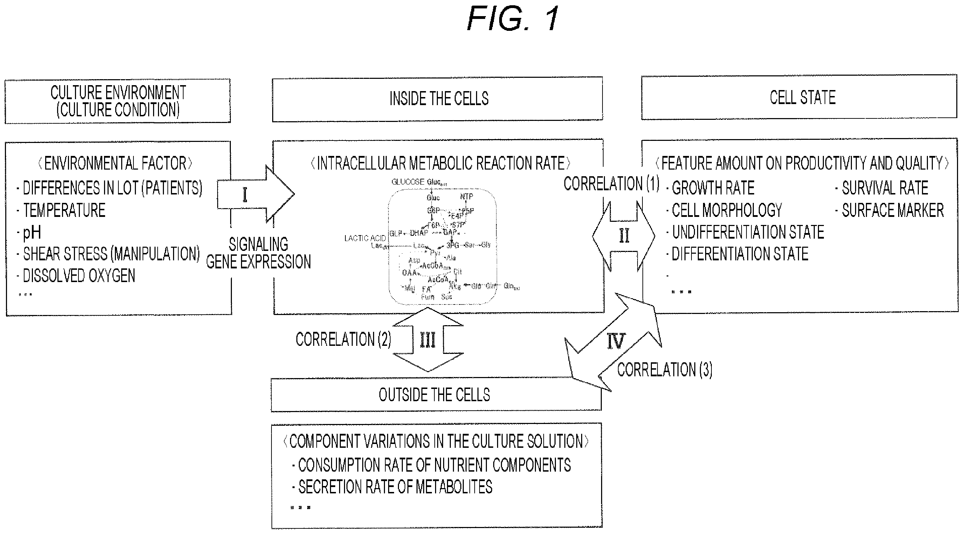

FIG. 1 shows a relationship among culture environment, intracellular metabolism, cell quality, and culture solution components. As shown in FIG. 1, when the culture environment of cells changes, in many cases, as a biochemical reaction, intracellular metabolism (each reaction rate in a metabolic pathway) changes (an arrow I in FIG. 1) via intracellular signaling, gene expression, and the like. Thus, the properties of the cells (quality such as cell type and productivity such as growth rate) change (an arrow II in FIG. 1). At the same time, the change in intracellular metabolism causes a change in the consumption rate of nutrient components by the cells and the secretion rate of waste products and the like to the outside of the cells (an arrow III in FIG. 1). Therefore, the consumption rate of nutrient components or the secretion rate of metabolites such as waste products reflects the cell state (an arrow IV in FIG. 1).

Therefore, with respect to changes in specific culture environment, a correlation between "the intracellular metabolic rate" and "the properties of the cells" (correlation (1)) and a correlation between "the intracellular metabolic rate" and "the consumption rate of nutrient components or the secretion rate of metabolites (time variations in component concentration in the culture solution) (correlation (2)) is acquired in advance, and a correlation between "the consumption rate of nutrient components or the secretion rate of metabolites" and "the properties of the cells" (correlation (3)) can be determined from the relationship between correlation (1) and correlation (2). Consequently, the properties of the cells can be determined from the time variations in extracellular component concentration in the culture solution. It is desirable to make a database (DB) of correlations (1) to (3) in advance based on culture experiments, and examples of the culture environment include differentiation inducing factors, temperature, pH, shear stress, dissolved oxygen, dissolved carbon dioxide, nutrient component concentration, waste product concentration, and the like.

As a procedure for determining correlation (3), the case of differentiation induction in human mesenchymal stem cells is mentioned. This result is an example of a cell state index in differentiation induction which has been revealed for the first time by the inventor's experiment. Although a specific procedure will be described in Examples, first, in order to allow human mesenchymal stem cells to differentiate into adipocytes, the medium is replaced with a differentiation inducing medium for adipocytes, and undifferentiated cells are gradually changed into adipocytes. When changes over time in intracellular metabolic reaction rate at the time of differentiation induction are examined by intracellular metabolic analysis, a specific intracellular metabolic pathway (part of glycolysis) correlates with the progression of differentiation of adipocytes (correlation (1)). In addition, when extracellular medium components at the time of differentiation induction are measured, there is a correlation between the concentration variation of alanine, serine, and glycine and the specific intracellular metabolic reaction rate (part of the glycolysis) (correlation (2)). Based on correlations (1) and (2), the progression of differentiation into adipocytes can be estimated using extracellular medium components (alanine, serine, and glycine) as indices. In Examples, amino acids and metabolites as indices of the induction of differentiation into osteoblasts and neuronal cells, in addition to differentiation into adipocytes, are found. By constructing and referring to the correlations as a metabolic correlation database, it is possible to estimate the cell state during culture from the component variation in the culture solution.

Specifically, the differentiation state of the cells can be evaluated by determining the progression of induced differentiation using any of the metabolites of glycolysis or any of the metabolites of the tricarboxylic acid cycle (TCA cycle), the metabolites being selected from among two or more types of amino acids contained in the culture solution or components in the culture solution derived from metabolism of the cells. In addition, a ratio of amounts of change over time in two or more types of amino acids contained in the culture solution, a ratio of amounts of change over time in any two or more types of the metabolites of glycolysis, or a ratio of amounts of change over time in any two or more types of the metabolites of the tricarboxylic acid cycle (TCA cycle) is preferably used as an index of progression of induced differentiation.

In the case of inducing differentiation into adipocytes, it is preferable to determine the progression of induced differentiation using any two or more types among alanine, serine, and glycine contained in the culture solution, or any of metabolites of glycolysis: glyceraldehyde-3-phosphate (GAP), 3-phosphoglyceric acid (3PG), and phosphoenolpyruvate (PEP).

In the case of inducing differentiation into osteoblasts, it is preferable to determine the progression of induced differentiation using valine and leucine contained in the culture solution, or metabolites of the tricarboxylic acid cycle (TCA cycle): α-ketoglutaric acid (AKG), succinic acid (Suc), and fumaric acid (Fum).

In the case of inducing differentiation into neuronal cells, it is preferable to determine the progression of induced differentiation using glycine and threonine contained in the culture solution, or metabolites of the tricarboxylic acid cycle (TCA cycle): α-ketoglutaric acid (AKG), succinic acid (Suc), and fumaric acid (Fum).

The cell culture system will be described below.

### [Cell Culture System]

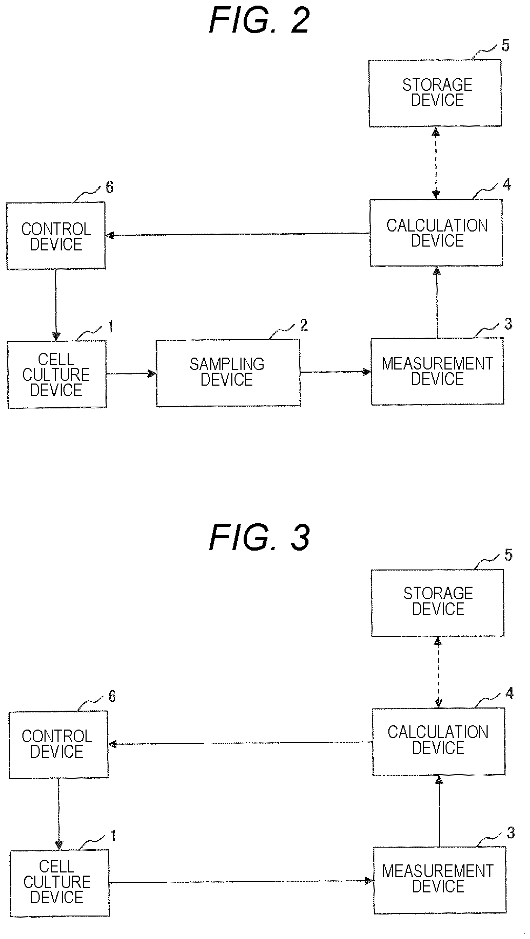

FIG. 2 illustrates a configuration diagram of a cell culture system according to an embodiment of the present invention. The cell culture system includes a cell culture device 1, a collection device (sampling device) 2 that collects a culture solution in the cell culture device, a measurement device 3 that measures an amount of a component in the culture solution, a storage device (data database) 5, a calculation device 4 that determines the progression of induced differentiation of the cells, and a control device 6 that controls the culture environment of the cell culture device.

The cell culture system is not limited to the configuration of FIG. 2, and any system may be used as long as the system can culture cells, monitor a specific component serving as an index in a culture solution, and determine the cell state based on the variation of the component.

FIG. 3 illustrates a configuration diagram of a cell culture system according to another embodiment of the present invention. For example, when a culture solution component analyzer can perform in-line measurement, it is possible to remove the collection device 2 as illustrated in FIG. 3.

Hereinafter, each device in FIG. 2 and a procedure of system operation will be described.

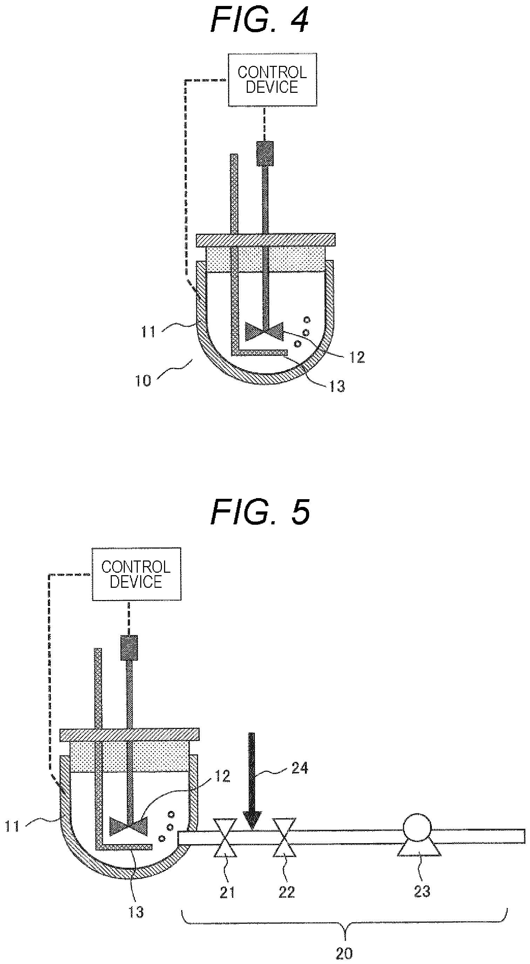

The cell culture device is a culture device for inducing differentiation of undifferentiated pluripotent stem cells into desired cells or causing the cells to glow. FIG. 4 illustrates an example of a cell culture device 10. The cell culture device includes various sensors for controlling culture environments, a heater 11 for controlling temperature, a stirring blade 12, and an aeration mechanism 13. The various sensors are sensors for pH, dissolved oxygen concentration (DO), thermocouple, and the like. The aeration mechanism is a mechanism for adding an alkali solution and aerating various gases (such as carbon dioxide, air, pure oxygen, and nitrogen) from the surface of the solution or from the inside of the solution in order to maintain the pH and the DO at set values.

The temperature of the culture solution in the culture tank is controlled to a desired value by turning on and off the heater 11 placed around the culture tank. In the case of suspension culture of cells, a stirring blade 12 is provided in the culture tank, and the inside of the culture tank is stirred by driving with a motor. In the differentiation induction, a differentiation inducing factor is added to the culture tank, or the growth medium is replaced with a differentiation inducing medium containing the differentiation inducing factor. In the medium replacement, in order to remove the growth medium, the cells and the medium can be separated by a method of separating the cells and the medium using a centrifuge, or filtration using a filter such as a hollow fiber membrane.

The collection device (sampling device) is a device that collects a culture solution from a cultured layer in the culture device. It is preferable that the culture solution can be sampled temporally and aseptically. FIG. 5 illustrates a sampling device 20. As illustrated in FIG. 5, a sampling pipe comes out of the culture tank, and is normally closed by valves 21 and 22. Immediately before the sampling of the culture solution, the valve 22 is opened, the inside of the pipe is sterilized with high-temperature steam from a steam line 24. After the temperature is sufficiently cooled, the valve 21 is opened and a pump is driven to extract the culture solution. After removing the necessary amount, the valve 21 is closed, the pump is stopped, cleaning with cleaning liquid and sterilization with high-temperature steam are performed, and the valve 22 is closed. The extracted culture solution is analyzed by a measurement device.

FIG. 5 illustrates a case where the pipe is made of a material resistant to heat, such as stainless steel. In the case of the pipe made of plastic, the number of plastic pipes necessary for sampling is prepared in advance, and the pipes and the culture tank are sterilized with gamma rays or the like. All the pipes with valves are closed when sampling is not performed. In the sampling, a valve of a pipe is opened, the culture solution is sampled by operating the pump, and the valve is closed after the end of sampling. In order to prevent contamination of bacteria, the pip used for sampling once is not used again. Alternatively, a method of aseptically replacing tubes using a sterile welding-cutting device may be employed.

The measurement device is a device that measures a component concentration of any of the metabolites of glycolysis or any of the metabolites of the tricarboxylic acid cycle (TCA cycle), the metabolites being selected from among two or more types of amino acids contained in the culture solution collected by the collection device or components in the culture solution derived from metabolism of the cells. In the case of at-line measurement, chromatography, liquid chromatograph mass spectrometer (LC-MS), gas chromatograph mass spectrometer (GC-MS), or the like can be used. In the case of in-line measurement, near infrared spectroscopy (NIR), Raman spectroscopy, or the like can be used. In the case of in-line measurement, the sampling device illustrated in FIG. 2 can be omitted, thereby simplifying the system (see FIG. 3). The device is not limited to the above-described spectrometers as long as the device analyzes the concentration of the medium component.

The storage device (database) stores information on changes over time in a culture solution component for each culture environment. The information on changes over time in a culture solution component for each culture environment is, for example, data on changes over time in alanine, serine, and glycine when inducing human mesenchymal stem cells to differentiate into adipocytes, data on changes over time in valine and leucine when inducing human mesenchymal stem cells to differentiate into osteoblasts, and data on changes over time in glycine and threonine when inducing human mesenchymal stem cells to differentiate into neuronal cells.

The calculation device determines the progression of induced differentiation from the information on changes over time in culture solution component stored in the storage device and the measurement results of the measurement device. The results analyzed by the measurement device are stored, variations of data on changes over time in culture solution component are calculated, and the cell state is determined with reference to the database. Preferably, the calculation device determines the progression of induced differentiation using a ratio of amounts of change over time in two or more types of amino acids contained in the culture solution, a ratio of amounts of change over time in any two or more types of the metabolites of glycolysis, or a ratio of amounts of change over time in any two or more types of the metabolites of the tricarboxylic acid cycle (TCA cycle) as an index of progression of differentiation.

For example, a ratio of changes over time (differences) in specific amino acids and changes over time in other amino acids is calculated. The changes over time in amino acids correlate with the consumption or secretion by the cells, and the influence of cell density can be offset by taking the ratio of variations of two different amino acids. Alternatively, a time integration ratio of changes over time (differences) in specific amino acids and changes over time in cell density may be calculated (corresponding to the consumption or secretion rate of amino acids per cell).

The control device is a device for controlling the culture environment of the cell culture device based on the progression of induced differentiation. For example, when the calculation device determines that differentiation induction ends, the calculation device generates an alert notifying that the process proceeds to the next step. Then, the process proceeds to the next purifying step. In a case where the differentiation induction is insufficient, the control of the culture environment is continued so as to maintain a constant level of the culture environment as it is. In a case where it is determined that the differentiation induction is abnormal, an alert indicating that the differentiation induction is abnormal is displayed. In a case where the differentiation induction can be returned to normal by changing the culture conditions, the set value of the culture conditions is changed. Meanwhile, in a case where the differentiation induction cannot be returned to normal, the control of interrupting the culture is performed.

The system is configured to include the above devices so that it is possible to automatically culture cells, and some of the above devices may be manually activated through aseptic operation.

### [Operation of System]

A method of operating the cell culture system (FIG. 2) is as follows.

Culture conditions are set by the cell culture device 1, and cells are seeded in the culture tank at the time when the culture environment is stabilized. The cells are allowed to grow. While monitoring the number of cells, when the number of cells reaches the desired number of cells, the growth medium is replaced with a differentiation inducing medium. Thereafter, the culture solution is periodically extracted using the sampling device 2 and analyzed by the measurement device 3. After the analysis results are appropriately calculated by the calculation device, the calculation results are collated with the database (DB) 5. In accordance with the results of the collation, the control of continuing the culture or ending the culture is performed, and the process proceeds to the next step. In the case of abnormality, the return to normal, the end of the culture, or the like is determined, and the control device takes appropriate responses.

### [Procedure of Determining Progression of Inducted Differentiation]

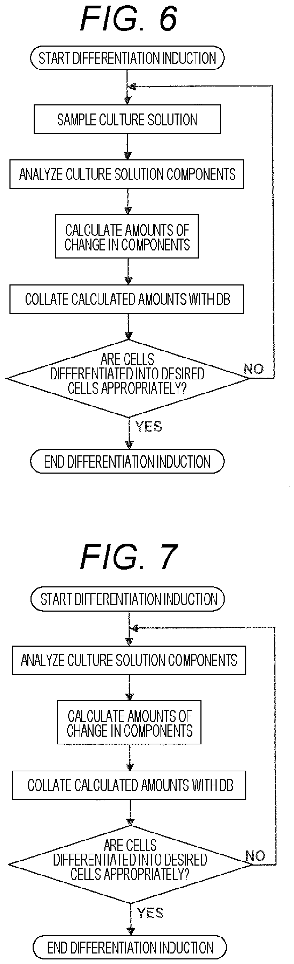

A determination procedure in the calculation device is shown in FIG. 6. After the growth of undifferentiated cells, the differentiation induction is started. Thereafter, the culture solution is aseptically sampled on a periodic basis, and the component concentration in the culture solution is measured. Changes over time in component concentration in the culture solution are calculated and collated with the database to determine the cell state. At this time, when the cells are differentiated into desired cells, the end of differentiation induction is displayed as an alert, and the culture is ended. When the cells are not differentiated into the desired cells, the culture is continued, and the above procedure is repeated until the cells are differentiated into the desired cells.

FIG. 7 shows a procedure for determining the progression of induction of cells in in-line monitoring. In the in-line monitoring, aseptic sampling can be omitted.

Hereinafter, the method for evaluating the progression of induced differentiation of cells will be described in detail with reference to Examples.

### Example 1

### Culture of Human Mesenchymal Stem Cells and Intracellular Metabolic Analysis

### < Reagents Used in Experiments >

For culture experiments, human mesenchymal stem cells (hMSCs) were purchased from PromoCell GmbH and used. A medium for growing mesenchymal stem cells 2 (PromoCell GmbH) was used for growth of mesenchymal stem cells, a medium for differentiation of mesenchymal stem cells into adipocytes 2 (PromoCell GmbH) was used for differentiation into adipocytes, a medium for differentiation of mesenchymal stem cells into osteoblasts (PromoCell GmbH) was used for differentiation into osteoblasts, and a medium for differentiation of mesenchymal stem cells into neuronal cells (PromoCell GmbH) was used for differentiation into neuronal cells. As a cell scaffold of fibronectin used at the time of differentiation induction, a fibronectin solution (human) (PromoCell GmbH) was used. In the experiment for metabolic analysis, for isotope labeling of cells, three types of U-13C glucose (Cambridge Isotope Laboratories, Inc.), 1-13C glucose (Cambridge Isotope Laboratories, Inc.), and natural glucose (added to the medium at the time of purchase) were added at a ratio of 1 : 1 : 2.

### < Preparation of Undifferentiated Stem Cells >

A human-derived fibronectin solution (10 µg/ml) was placed in a 6-well plate at 1 ml/well, and plate coating was performed at room temperature for 1 hour. After removal of the fibronectin solution, the hMSCs were adjusted to a predetermined cell density using the medium for growing mesenchymal stem cells 2, 2 ml of the cell solution was added to each well, and the cells were cultured in a 5% CO₂ incubator at 37°C (differentiation into adipocytes and differentiation into osteoblasts: 1 × 10⁵ cells/well, differentiation into neuronal cells: 4 × 10⁴ cells/well).

After the cells reached a predetermined density (in the case of differentiation into adipocytes: 80 to 90% confluent, in the case of differentiation into osteoblasts: 100% confluent, in the case of differentiation into neuronal cells: 60 to 80% confluent), the medium was replaced with each of the differentiation inducing media (the medium for growing mesenchymal stem cells 2, the medium for differentiation into adipocytes, or the medium for differentiation into osteoblasts). Medium replacement was performed once every 3 days. At the time of medium replacement, the supernatant of the culture solution before replacement was recovered, and amino acid analysis was performed at a later date. In addition, sample preparation for metabolic analysis was also performed at the time of medium replacement.

### < Preparation of Cell Sample for Intracellular Metabolic Analysis >

The 6-well plate in which the cells were isotope-labeled was taken out from the incubator and washed once with a phosphate buffer solution. The phosphate buffer solution was removed, and then 200 µL of methanol cooled to -20°C was added and spread over all the wells. The plate was transferred on ice, and 600 µL of distilled water was added to each of the wells. The cells were peeled off by rubbing the well surface with the tip of a Pipetman chip, and transferred to an ice-cooled microtube. The microtube was subjected to ultrasonic treatment for 1 minute, and then 800 µL of chloroform was added thereto. The mixture was mixed in a vortex mixer at 4°C for 30 minutes, centrifuged in a centrifuge (at 11,500 rpm and 4°C for 30 minutes), and the upper layer divided into two layers was transferred to another microtube. The upper layer was dried by evaporation overnight.

30 µL of 2% methoxyamine hydrochloride (Pierce) was added to the dried sample, and the mixture was gently mixed by vortex. The solution was collected in the lower part by tabletop centrifugation, followed by reaction on a heat block at 37°C for 2 hours. 45 µL of MTBSTFA (N-tertButyldimethylsilyl-N-methyltrifluoroacetamide) + 1% TBDMCS (tert-butyldimethylchlorosilane) solution (Pierce) was added, and the mixture was gently mixed by vortex. The solution was collected in the lower part by tabletop centrifugation, followed by reaction on a heat block at 55°C for 1 hour. The reaction solution was transferred into a container for GC/MS analysis, and stored at room temperature until analysis.

In the GC/MS analysis, Agilent 6890 (Agilent Technologies, Inc.) and a 30 m DB-35MS capillary column were used. The measurement conditions were set such that the temperature was raised from 100°C to 300°C at 3.5°C/min, the inlet temperature was 270°C, the carrier gas was helium gas, and the analysis was performed at a flow rate of 1 mL/min.

### < Simulation of Intracellular Metabolic Analysis >

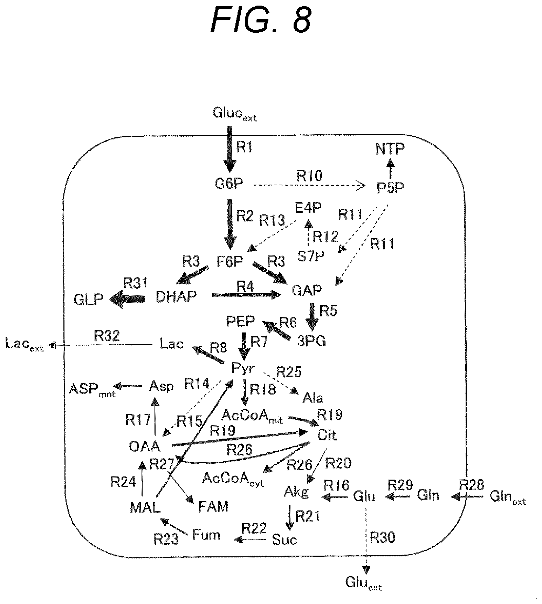

In the simulation, it is necessary to set an intracellular metabolic pathway of human mesenchymal stem cells to be analyzed. In this example, in order to perform intracellular metabolic analysis of animal cells, a metabolic pathway model generally used in animal cells shown in FIG. 8 was used.

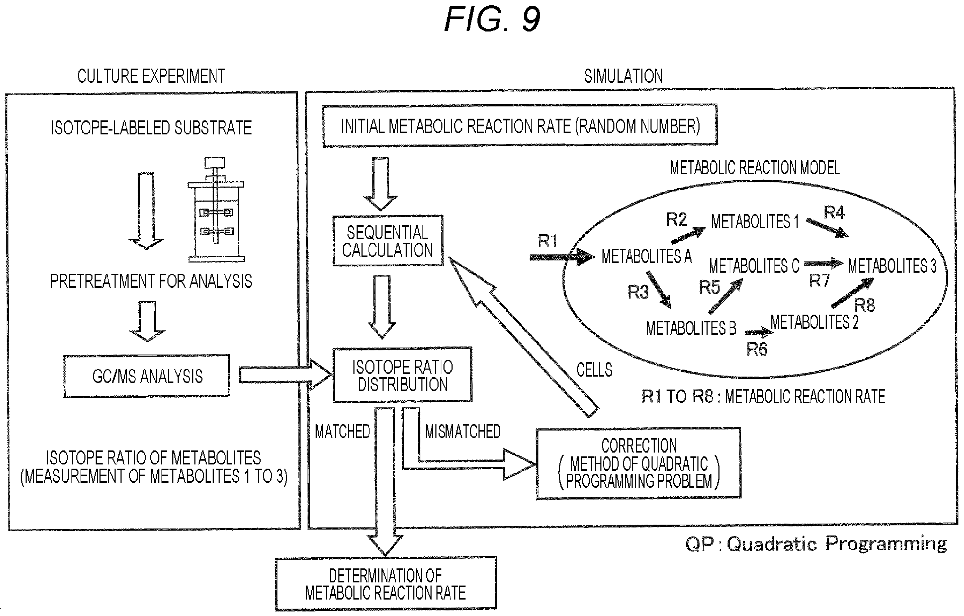

The concept of the method for estimating metabolic flux is shown in FIG. 9. In the simulation, random metabolic flux values (R1 to R8 in FIG. 9) are first given, and the ratio of the number of isotopic carbons contained in respective metabolites in the cells at a steady state is calculated based on the metabolic pathway model. When this calculated value is compared with the ratio of the number of isotopic carbons in the intracellular metabolites measured in the experiment, and there is no statistically significant difference, the value is determined as an estimated metabolic flux. When there is a statistically significant difference (when the calculated value is different from the ratio.), the metabolic flux values according to the simulation are corrected to minimize the mean square error between the value of the ratio of the number of isotopic carbons in the metabolites according to the simulation and the value of the ratio of the number of isotopic carbons in the metabolites according to the experiment.

The operation of calculating the ratio of the number of isotopic carbons with the modified metabolic flux and comparing the values of the ratio of the number of isotopic carbons in the experimental metabolites until there is no statistically significant difference is repeated. Usually, it can be estimated by repeating the calculation two or three times. When a statistically significant difference occurs even after repeating the calculation many times, it is determined that the metabolic pathway model is incorrect or the experimental data is not appropriately measured.

In the above simulation, observation parameters (the ratio of the number of isotopic carbons in the metabolites in the cells and extracellular metabolic flux) based on the experiments are required. Usually, the required observation parameters vary depending on the target cell, the medium to be used, or the like. In this example, regarding 11 types of intracellular metabolites: ALA, GLY, VAL, SER, ASP, GLU, MET, THR, ILE, Lac, and PYR, the fragmented substances (19 types) were used as input parameters of simulation, and the intracellular metabolic reaction rate was calculated.

### Example 2

### Determination of Progression of Differentiation in Induction of Differentiation into Adipocytes

According to Example 1, hMSCs in an undifferentiated state were cultured on a 6-well plate for 3 days. Thereafter, the medium was replaced with an isotope-labelled glucose-containing medium for differentiation into adipocytes, and the induction of differentiation into adipocytes was performed. The observation of cell morphology and metabolic analysis simulation were performed on the cells on day 3, day 6, day 9, day 12, and day 15 after the differentiation induction.

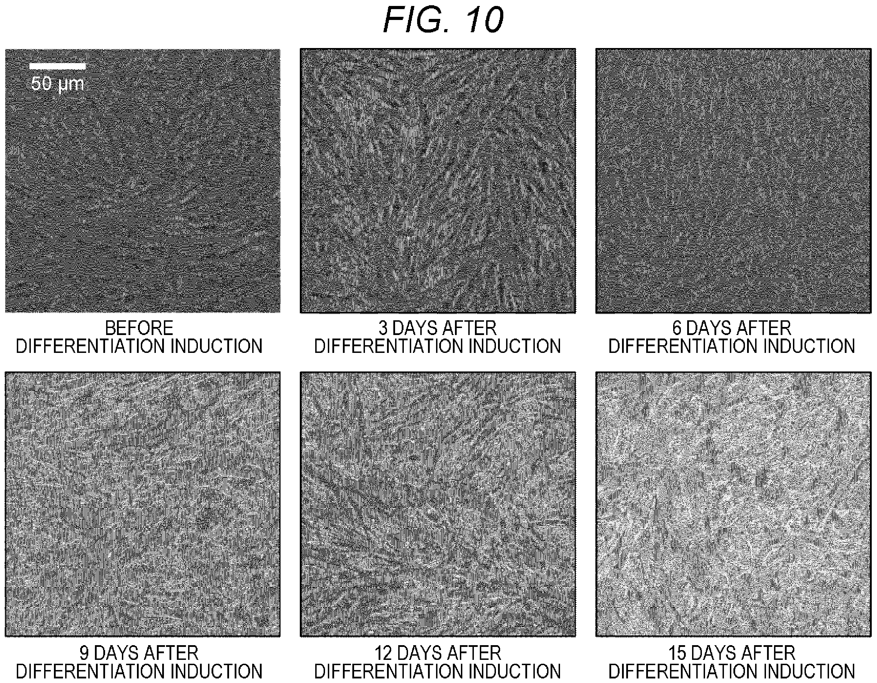

FIG. 10 is phase-contrast images showing changes over time in cell morphology after differentiation induction. Although the cells were elongated spindle-shaped cells until the sixth day of differentiation induction, lipid droplets, i.e., characteristics of adipocytes, were observed in the cytoplasm on the ninth day of differentiation induction. Thereafter, the lipid droplets increased. This indicates that differentiation into adipocytes progresses over time.

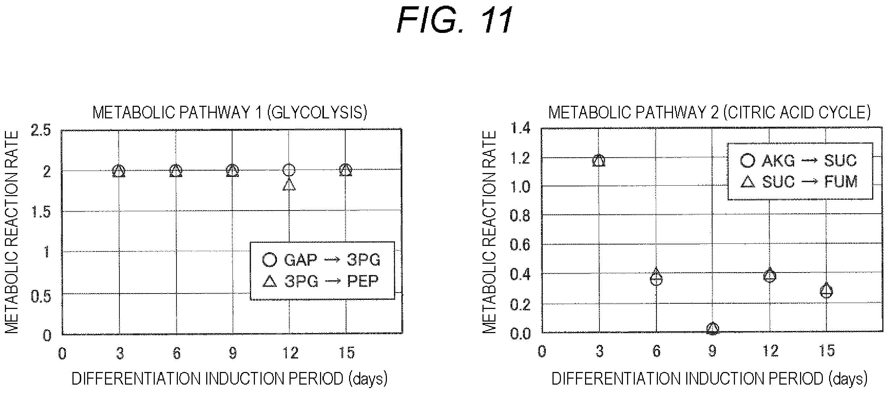

In order to examine correlation (1) in FIG. 1, changes over time in intracellular metabolic reaction rate were measured by the intracellular metabolic analysis shown in Example 1. As shown in FIG. 11, changes over time were not observed in part of glycolysis: GAP → 3PG → PEP (metabolic pathway 1), and the result was constant. However, the metabolic reaction rate of part of the citric acid cycle: AKG → SUC → FUM (metabolic pathway 2) decreased until the ninth day of differentiation induction. Thereafter, the metabolic reaction rate slightly increased, and a stable correlation was observed until the fifteenth day of differentiation induction. The other metabolic pathways did not correlate with the progress of induced differentiation.

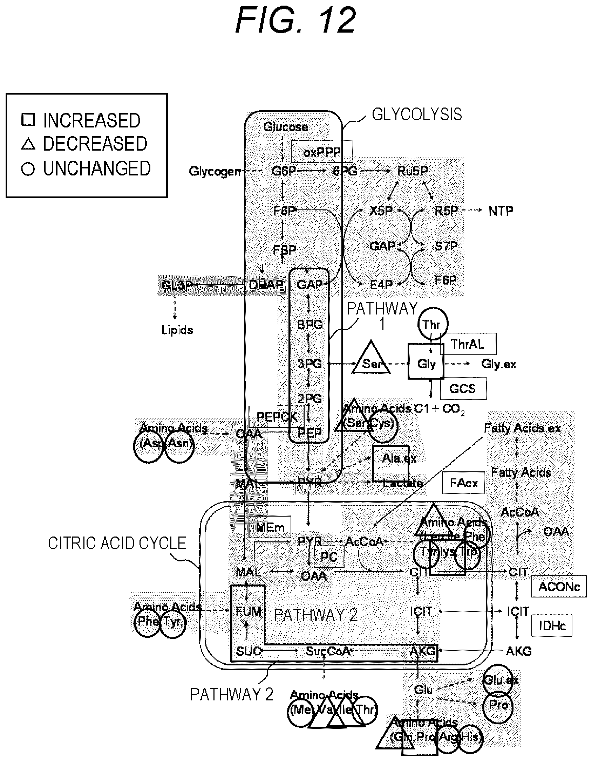

Next, in order to examine correlation (2) in FIG. 1, changes over time in amino acid concentration in the culture solution were measured. FIG. 12 shows the relationship between the intracellular metabolic reaction pathways and the amino acids secreted to the culture solution. As a result of measuring the concentrations of the amino acids in the culture solution over time, the increased amino acids in the culture solution with progression of the induction of differentiation into adipocytes were surrounded by squares, the decreased amino acids were surrounded by triangles, and the unchanged amino acids were surrounded by circles. The reason why there is a change in concentration even in the amino acid in the vicinity of metabolic pathway 1 in which no change over time was observed is that there is a differentiation-induction-dependent pathway that is not considered in this simplified metabolic reaction pathway. There are three types of amino acids that change near metabolic pathway 1: alanine (Ala), glycine (Gly), and serine (Ser) .

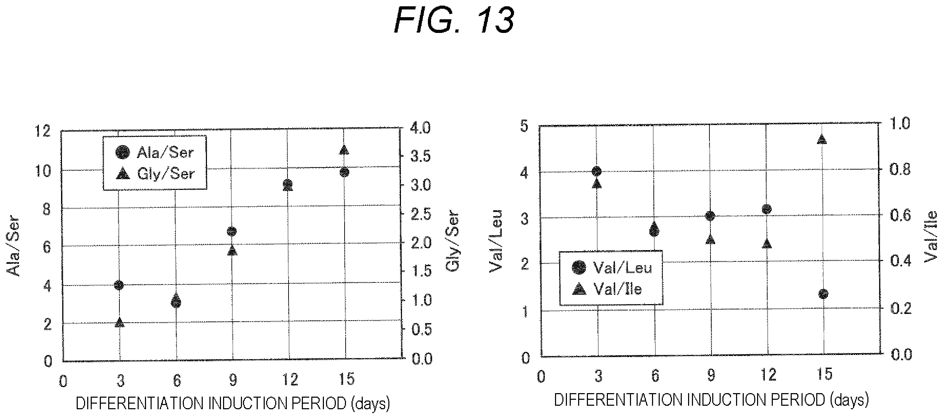

When the change in amino acid concentration is used as an index, the change in one type of amino acid concentration change is influenced by the cell number density and the like, and it is difficult to grasp the correct cell state from the value of the change in concentration. Therefore, it is desirable to select two or more types of amino acids, cancel the influence of the cell number density by the ratio, and then perform the evaluation. When seeing changes over time in the ratio of the concentration variations of each of the amino acids in FIG. 13 (a), and it is found that the value of the ratio constantly increases in accordance with differentiation induction. Meanwhile, amino acids that have changed in the vicinity of metabolic pathway 2 are valine (Val), leucine (Leu), isoleucine (Ile), and the like. As shown in the graph of FIG. 13 (b), changes over time in the ratio of these amino acids do not show a certain increase or decrease (have no correlation), and thus it is found to be difficult to use the changes over time in the ratio as the progress of induced differentiation. This is considered to be because other external factors (nutrient concentration and the like) except differentiation induction act as perturbations in metabolic pathway 2.

As described above, it is difficult to directly see correlation (3) even when measuring the progress of induced differentiation and the concentration variations in amino acids in the culture solution, and it is necessary to derive correlation (3) based on the scientific evidence via a biochemical model of cells (in this example, intracellular metabolic reaction rate).

From the above examination of correlation (1) and correlation (2), alanine, glycine, and serine in the culture solution correlate with the differentiation of human mesenchymal stem cells into adipocytes (correlation (3)), and these correlations can be used as index components.

### Example 3

### Determination of Progression of Differentiation in Induction of Differentiation into Osteoblasts

According to Example 1, hMSCs in an undifferentiated state were cultured on a 6-well plate for 3 days. Thereafter, the medium was replaced with an isotope-labelled glucose-containing medium for differentiation into osteoblasts, and the induction of differentiation into osteoblasts was performed. The observation of cell morphology and metabolic analysis simulation were performed on the cells on day 3, day 6, day 9, day 12, and day 15 after the differentiation induction.

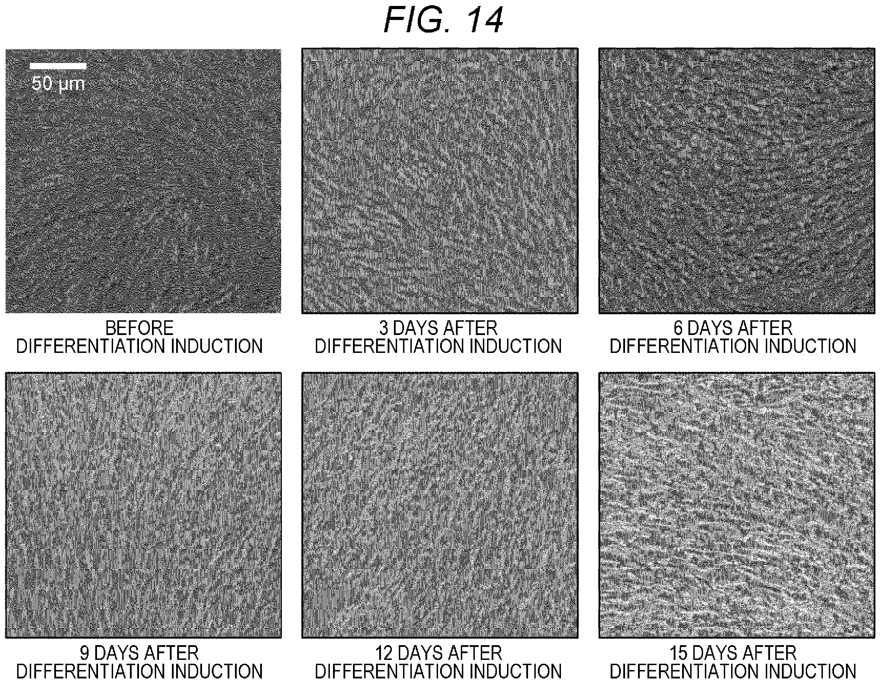

FIG. 14 is phase-contrast images showing changes over time in cell morphology after differentiation induction. The cells are elongated spindle-shaped cells until the third day of differentiation induction, but the cells form a convolutional pattern on the sixth day of differentiation induction. This indicates that differentiation into osteoblasts gradually progresses.

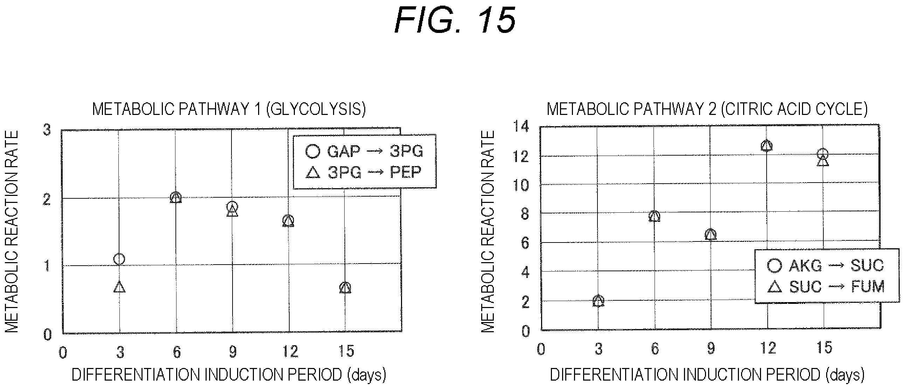

In order to examine correlation (1) in FIG. 1, changes over time in intracellular metabolic reaction rate were measured by the intracellular metabolic analysis shown in Example 1. As shown in FIG. 15, the results show that the value of part of glycolysis: GAP → 3PG → PEP (metabolic pathway 1) was varied unstably and did not depend on the progression of induced differentiation, whereas part of the citric acid cycle: AKG → SUC → FUM (metabolic pathway 2) was stable over time, and the metabolic reaction rate increased and depended on the progression of induced differentiation.

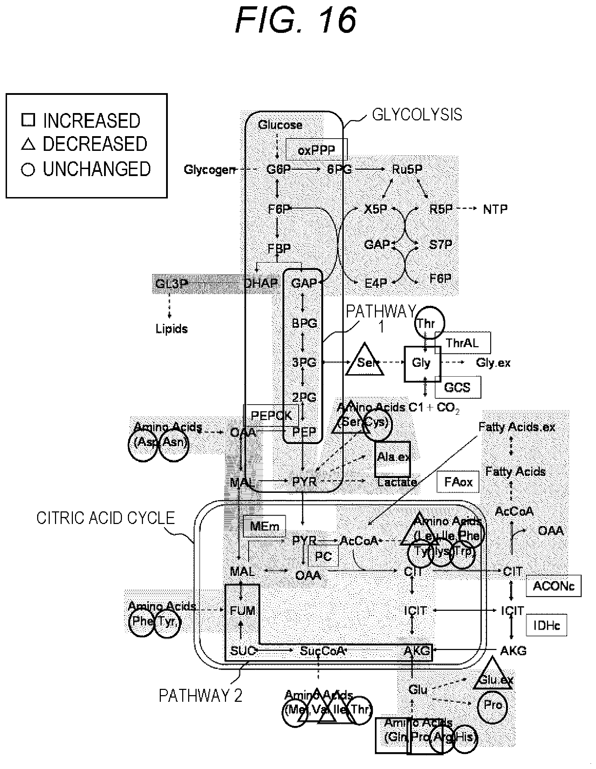

Next, in order to examine correlation (2) in FIG. 1, changes over time in amino acid concentration in the culture solution were measured. FIG. 16 shows the relationship between the intracellular metabolic reaction pathways and the amino acids secreted to the culture solution. As a result of measuring the concentrations of the amino acids in the culture solution over time, the increased amino acids in the culture solution with progression of the induction of differentiation into adipocytes were surrounded by squares, the decreased amino acids were surrounded by triangles, and the unchanged amino acids were surrounded by circles. As shown in FIG. 16, the concentrations of the amino acids in the culture solution at the time of differentiation induction were confirmed by an increase or decrease in various amino acids. An increase or decrease was observed in some of the amino acids in the vicinity of metabolic pathway 1 in which the value was varied unstably. However, since the metabolic reaction rate itself was unstable (did not depend on differentiation induction), it was determined that it was not appropriate to select as an index from the amino acids in the vicinity of metabolic pathway 1.

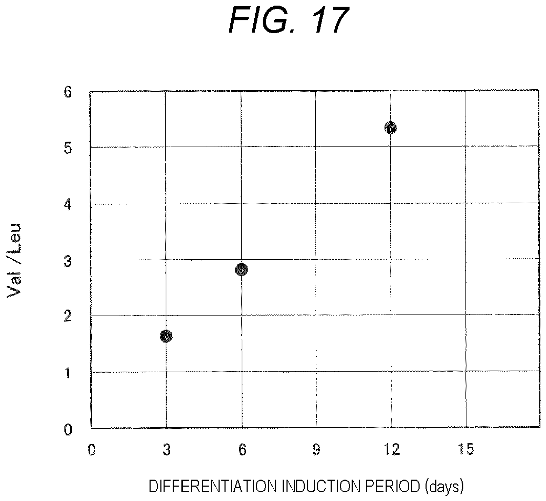

Meanwhile, valine (Val), leucine (Leu), and the like are observed as amino acids changed in the vicinity of metabolic pathway 2. It is found that when changes over time in the ratio of the amino acids are taken as an index candidate, the ratio increases over time as shown FIG. 17. The metabolic reaction is accelerated, whereas the index value decreases. This is caused by the large consumption of valine. Regarding other amino acids (proline (Pro) and the like), the concentration variation range is small, and the change of the index value is likely to vary, but the index value can be used.

From the above examination of correlation (1) and correlation (2), valine and leucine in the culture solution correlate with the differentiation of human mesenchymal stem cells into osteoblasts (correlation (3)), and these correlations can be used as index components.

### Example 4

### Determination of Progression of Differentiation in Induction of Differentiation into Neuronal Cells

According to Example 1, hMSCs in an undifferentiated state were cultured on a 6-well plate for 3 days. Thereafter, the medium was replaced with an isotope-labelled glucose-containing medium for differentiation into neuronal cells, and the induction of differentiation into neuronal cells was performed. The observation of cell morphology and metabolic analysis simulation were performed on the cells on day 3, day 6, day 9, day 12, and day 15 after the differentiation induction.

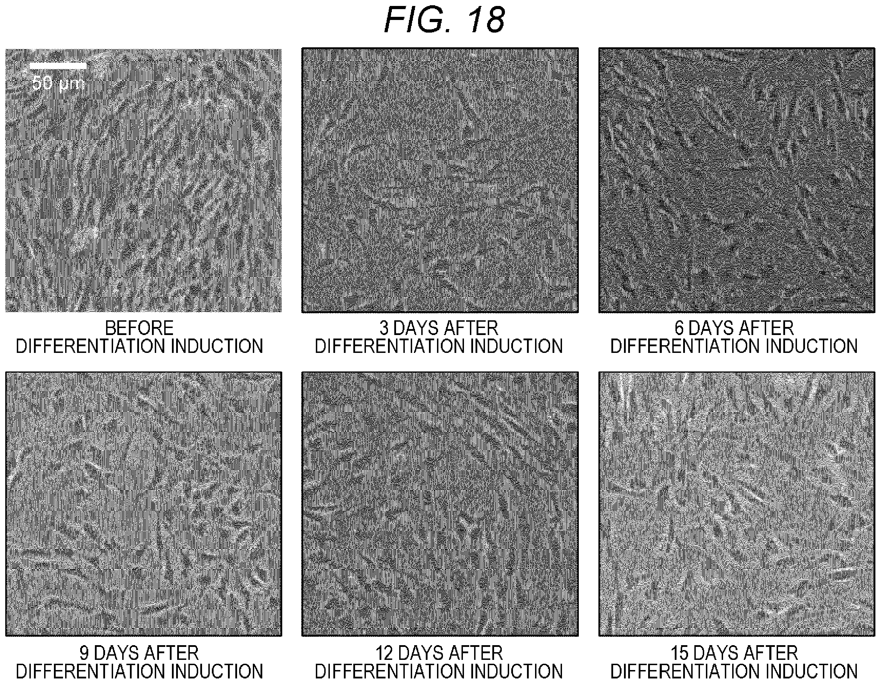

FIG. 18 is phase-contrast images showing changes over time in cell morphology after differentiation induction. On the third day of differentiation induction, it is found that the cells are differentiated into dendritic neuronal cells.

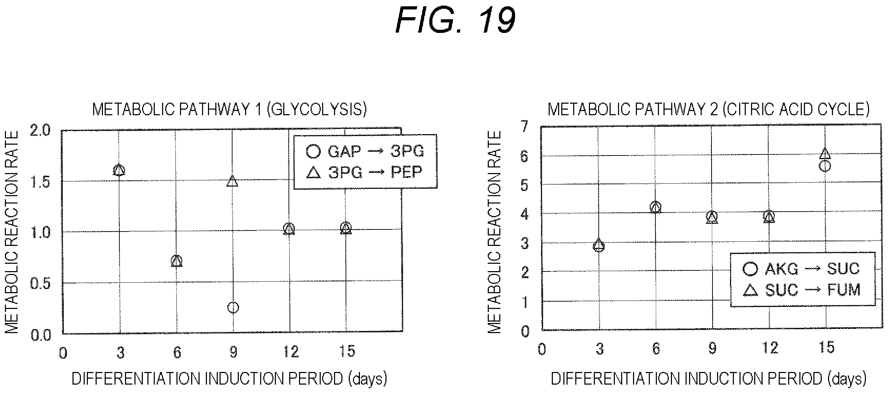

In order to examine correlation (1) in FIG. 1, changes over time in intracellular metabolic reaction rate were measured by the intracellular metabolic analysis shown in Example 1. As shown in FIG. 19, the results show that the value of part of glycolysis: GAP → 3PG → PEP (metabolic pathway 1) was varied unstably, whereas it is found that part of the citric acid cycle: AKG → SUC → FUM (metabolic pathway 2) was stable over time, and the metabolic reaction rate slightly increased.

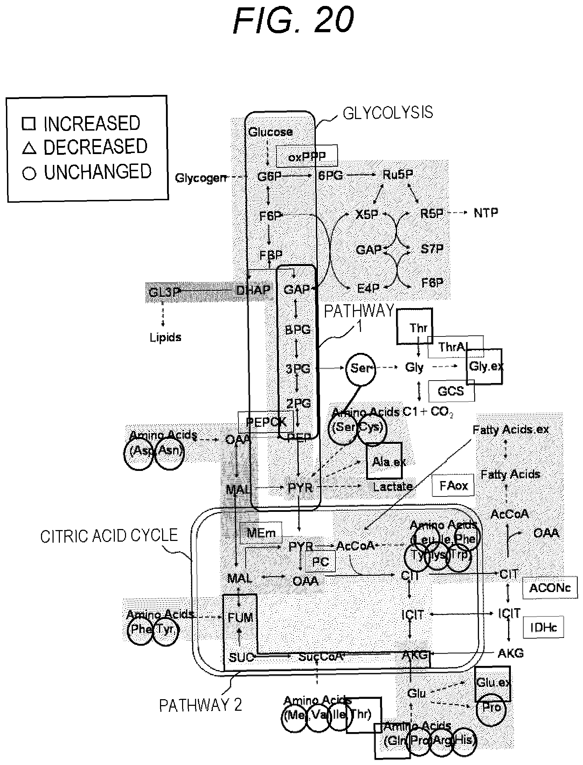

Next, in order to examine correlation (2) in FIG. 1, changes over time in amino acid concentration in the culture solution were measured. FIG. 20 shows the relationship between the intracellular metabolic reaction pathways and the amino acids secreted to the culture solution. As a result of measuring the concentrations of the amino acids in the culture solution over time, the increased amino acids in the culture solution with progression of the induction of differentiation into adipocytes were surrounded by squares, the decreased amino acids were surrounded by triangles, and the unchanged amino acids were surrounded by circles. As shown in FIG. 20, the concentrations of the amino acids in the culture solution at the time of differentiation induction were confirmed by an increase or decrease in various amino acids.

An increase was observed in some of the amino acids in the vicinity of metabolic pathway 1 in which the value was varied unstably. However, since the metabolic reaction rate itself was unstable, it was determined that it was not appropriate to select as an index from the amino acids in the vicinity of metabolic pathway 1, similarly to the case of osteoblasts. Meanwhile, glycine (Gly), threonine (Thr), and the like are observed as amino acids changed in the vicinity of metabolic pathway 2. It is found that when changes over time in the ratio of the amino acids are taken as an index candidate, the ratio increases over time. Glycine and threonine are also present in the vicinity of metabolic pathway 1. Since the metabolic reaction rate of metabolic pathway 2 is larger than that of metabolic pathway 1, it is considered that the influence of variation in the value of metabolic pathway 1 is small.

From the above examination of correlation (1) and correlation (2), glycine and threonine in the culture solution correlate with the differentiation of human mesenchymal stem cells into neuronal cells (correlation (3)), and these correlations can be used as index components.

### Example 5

Discrimination between Differentiated Culture and Undifferentiated Maintenance Culture in Differentiation Induction Index Value

In Examples 2 to 4, indices of adipocytes, osteoblasts, and neuronal cells were determined. In Example 5, examination is performed to confirm whether these index values can discriminate between the growth of differentiated cells and the growth of undifferentiated cells.

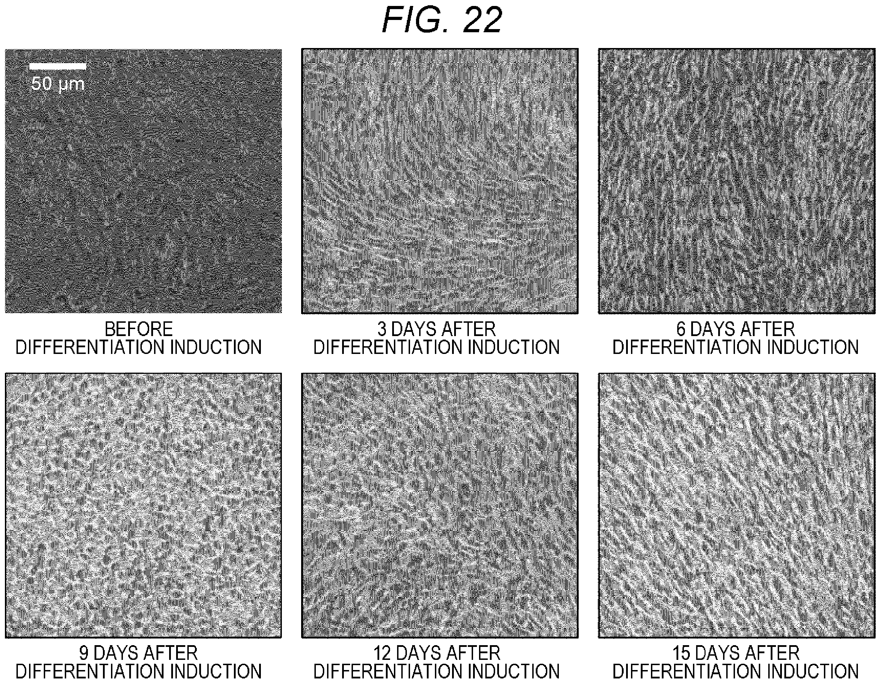

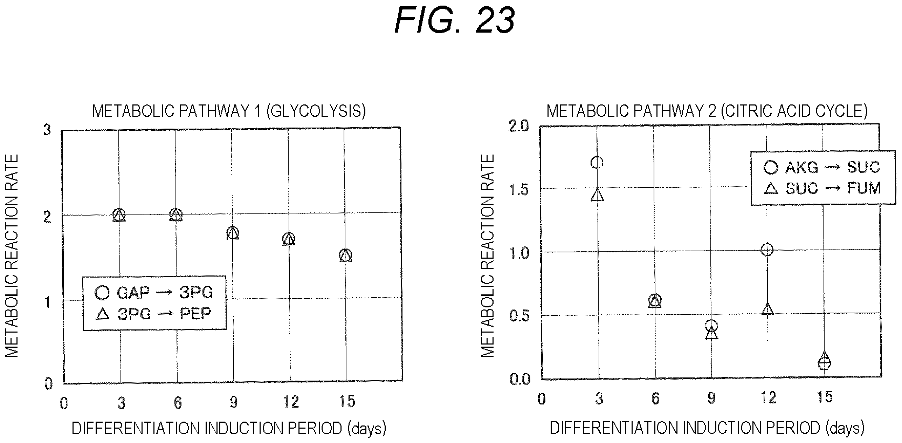

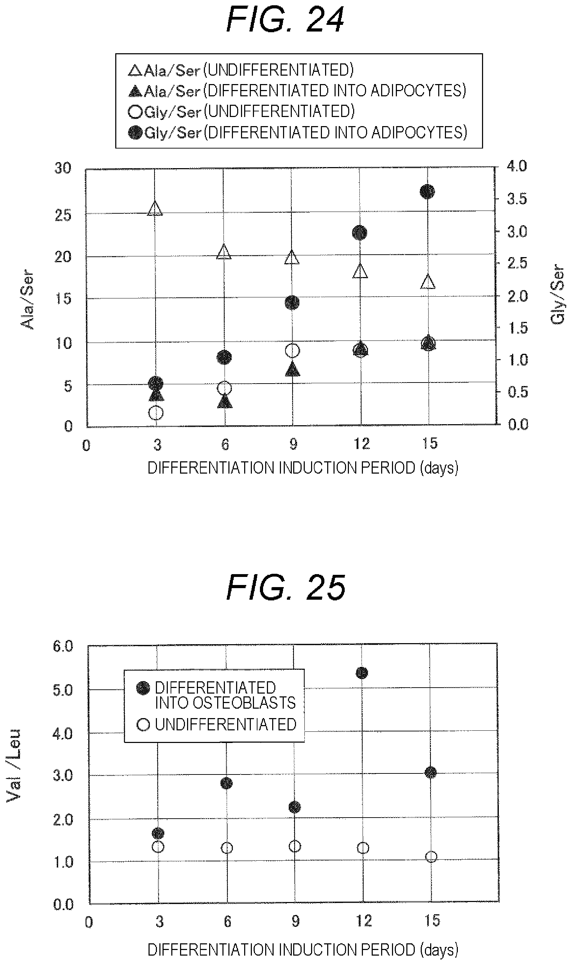

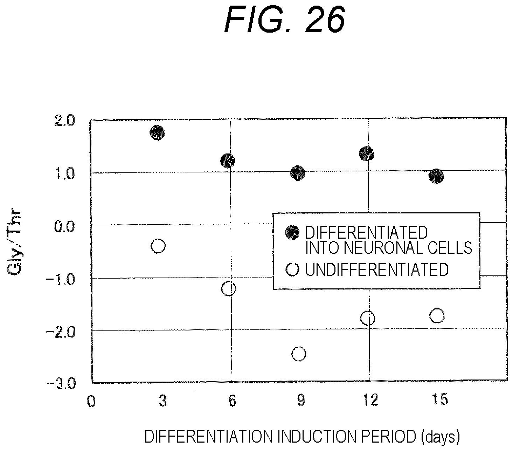

First, when the cells were grown in a state where the undifferentiation was maintained, fusiform cells became dense as shown in FIG. 22. The metabolic simulation is performed in this growing process, as a result of which it is found that the metabolic reaction rate changes over time even in the undifferentiated state as shown in FIG. 23. Therefore, in the growth of the cells in the undifferentiated state, the index value was calculated using the indices determined in each differentiation induction. The results are shown in FIGS. 24 to 26. As shown in FIG. 24, the index value (alanine/serine) of adipocytes increases in the case of adipocytes and decreases in the case of undifferentiated cells, thereby allowing for discrimination between them. In addition, in the case of the index value (glycine/serine), there is a large difference in variation range, thereby allowing for discrimination between them. In the case of osteoblasts, as shown in FIG. 25, the index value (valine/leucine) increases in differentiation induction, and shows no change in undifferentiation. In the case of neuronal cells, as shown in FIG. 26, the index value (glycine/threonine) takes a positive value in differentiation induction, whereas the index value takes a negative value in undifferentiation, thereby allowing for discrimination between them.

From the above results, it was confirmed that adipocytes, osteoblasts, neuronal cells, and undifferentiated cells were able to be discriminated from one another by the index values determined in this example.

The present invention is not limited to the examples described above, and various modified examples are included. For example, the above-described examples have been described in detail in order to explain the present invention in an easy-to-understand manner, and are not necessarily limited to those having all the configurations described. In addition, it is possible to replace a part of the configuration of a certain example with a configuration of other examples, and it is also possible to add a configuration of other examples to the configuration of a certain configuration. Furthermore, it is possible to add, eliminate, and replace other configurations with respect to a part of the configuration of each of the examples.

### [Abbreviations]

- AcCoA: Acetyl-CoA
- AKG: α-Ketoglutarate
- Ala: Alanine
- Asp: Aspartic acid
- Cit: Citric acid
- DHAP: Dihydroxyacetone phosphate
- E4P: Erythrose-4-phosphate
- Fum: Fumarate
- F6P: Fructose-6-phosphate
- GAP: Glyceraldehyde-3-phosphate
- Gln: Glutamine
- Glnext: Extracellular Glutamine
- Gluc: Glucose
- Glucext: Extracellular Glucose
- Glu: Glutamic acid
- Gly: Glycine
- G6P: Glucose-6-phosphate
- Lac: Lactic acid
- Lacext: Extracellular Lactic acid
- Mal: Malate
- Oac: Oxaloacetate
- PEP: Phosphoenolpyruvic acid
- Pyr: Pyruvate
- R5P: Ribose-5-phosphate
- Ser: Serine
- Suc: Succinate
- SucCoA: Succinyl-CoA
- S7P: Sedoheptulose-7-phosphate
- Thr: Threonine
- 3PG: 3-phosphoglycerate

### Reference Signs List

- 1: cell culture device
- 2: sampling device
- 3: measurement device
- 4: calculation device
- 5: storage device
- 6: control device
- 10: culture tank
- 11: heater
- 12: stirring blade
- 13: aeration mechanism
- 20: sampling device
- 21: valve 1
- 22: valve 2
- 23: pump
- 24: steam line

## Claims

1. A method for evaluating a differentiation state of cells during culturing for inducing undifferentiated pluripotent stem cells to differentiate into desired cells, wherein the method determines the progression of induced differentiation using any of the metabolites of glycolysis or any of the metabolites of the tricarboxylic acid cycle (TCA cycle), the metabolites being selected from among two or more types of amino acids contained in the culture solution or components in the culture solution derived from metabolism of the cells.

2. The method for evaluating a differentiation state of cells according to claim 1, wherein a ratio of amounts of change over time in two or more types of amino acids contained in the culture solution, a ratio of amounts of change over time in any two or more types of the metabolites of glycolysis, or a ratio of amounts of change over time in any two or more types of the metabolites of the tricarboxylic acid cycle (TCA cycle) is used as an index of progression of induced differentiation.

3. The method for evaluating a differentiation state of cells according to claim 1 or 2, wherein
when the desired cells are adipocytes, the progression of induced differentiation is determined using any two or more types among alanine, serine, and glycine contained in the culture solution.

4. The method for evaluating a differentiation state of cells according to claim 1 or 2, wherein
when the desired cells are osteoblasts, the progression of induced differentiation is determined using valine and leucine contained in the culture solution.

5. The method for evaluating a differentiation state of cells according to claim 1 or 2, wherein
when the desired cells are neuronal cells, the progression of induced differentiation is determined using glycine and threonine contained in the culture solution.

6. The method for evaluating a differentiation state of cells according to claim 1 or 2, wherein when the desired cells are adipocytes, the progression of induced differentiation is determined using any of glyceraldehyde-3-phosphate (GAP), 3-phosphoglyceric acid (3PG), and phosphoenolpyruvate (PEP) as the metabolites of glycolysis.

7. The method for evaluating a differentiation state of cells according to claim 1 or 2, wherein when the desired cell are osteoblasts, the progression of induced differentiation is determined using any of α-ketoglutaric acid (AKG), succinic acid (Suc), and fumaric acid (Fum) as the metabolites of the tricarboxylic acid cycle (TCA cycle).

8. The method for evaluating a differentiation state of cells according to claim 1 or 2, wherein when the desired cell are neuronal cells, the progression of induced differentiation is determined using any of α-ketoglutaric acid (AKG), succinic acid (Suc), and fumaric acid (Fum) as the metabolites of the tricarboxylic acid cycle (TCA cycle).

9. A cell culture system comprising:
a cell culture device that induces undifferentiated pluripotent stem cells to differentiate into desired cells;
a collection device that collects a culture solution in the cell culture device;
a measurement device that measures an amount of any of the metabolites of glycolysis or any of the metabolites of the tricarboxylic acid cycle (TCA cycle), the metabolites being selected from among two or more types of amino acids contained in the culture solution collected by the collection device or components in the culture solution derived from metabolism of the cells;
a storage device that stores information on changes over time in a culture solution component for each culture environment; and
a calculation device that determines progression of induced differentiation from the information on changes over time in the culture solution component stored in the storage device and measurement results of the measurement device.

10. The cell culture system according to claim 9, wherein the calculation device determines the progression of induced differentiation using a ratio of amounts of change over time in two or more types of the amino acids, a ratio of amounts of change over time in any two or more types of the metabolites of glycolysis, or a ratio of amounts of change over time in any two or more types of the metabolites of the tricarboxylic acid cycle (TCA cycle) as an index of progression of differentiation.

11. The cell culture system according to claim 9 or 10, further comprising a control device that controls a culture environment of the cell culture device based on the progression of induced differentiation.
